# EUROPÄISCHE PATENTSCHRIFT

(11) **EP 1 636 350 B1**
(45) Veröffentlichungstag und Bekanntmachung des Hinweises auf die Patenterteilung: **12.09.2007**
(21) Anmeldenummer: 04740218.5
(22) Anmeldetag: 23.06.2004
(51) Int. Cl.: C12N 5/06, A61K 35/39

(54) **VERFAHREN ZUR DIFFERENZIERUNG VON STAMMZELLEN IN ZELLEN, DIE EIN PANKREATISCHES HORMON PRODUZIEREN**
METHOD FOR DIFFERENTIATING STEM CELLS IN CELLS THAT PRODUCE A PANCREATIC HORMONE
PROCEDE POUR DIFFERENCIER DES CELLULES SOUCHES EN CELLULES QUI PRODUISENT UNE HORMONE PANCREATIQUE

(30) Priorität: 23.06.2003 DE 10328280; 08.04.2004 DE 102004017473
(43) Veröffentlichungstag der Anmeldung: 22.03.2006
(73) Patentinhaber: Fraunhofer-Gesellschaft zur Förderung der angewandten Forschung e.V., 80686 München (DE)
(72) Erfinder: KRUSE, Charli, 23923 Herrnburg (DE); FUHR, Günter, 13187 Berlin (DE)
(74) Vertreter: Katzameyer, Michael
(86) Internationale Anmeldenummer: PCT/EP2004/006799
(87) Internationale Veröffentlichungsnummer: WO 2005/001073

(56) Entgegenhaltungen:
- WO-A-00/78929
- WO-A-02/059278
- WO-A-02/086107
- BONNER-WEIR ET AL: "In vitro cultivation of human islets from expanded ductal tissue" PROCEEDINGS OF THE NATIONAL ACADEMY OF SCIENCES OF USA, NATIONAL ACADEMY OF SCIENCE. WASHINGTON, US, Bd. 97, Nr. 14, 5. Juli 2000 (2000-07-05), Seiten 7999-8004, XP002144480 ISSN: 0027-8424
- RAMIYA VIJAYAKUMAR K ET AL: "Reversal of insulin-dependent diabetes using islets generated in vitro from pancreatic stem cells" NATURE MEDICINE, NATURE AMERICA, NEW YORK, US, Bd. 6, Nr. 3, März 2000 (2000-03), Seiten 278-282, XP000864764 ISSN: 1078-8956
- SORIA B: "In-vitro differentiation of pancreatic beta-cells" DIFFERENTIATION, SPRINGER VERLAG, DE, Bd. 68, Nr. 4-5, Oktober 2001 (2001-10), Seiten 205-219, XP002229903 ISSN: 0301-4681

## Beschreibung

Die vorliegende Erfindung betrifft Verfahren zum Differenzieren von Stammzellen in Zellen, die ein pankreatisches Hormon produzieren, insbesondere in Insulin-produzierende Zellen, pankreatisches Hormon produzierende Zellen, Zellzusammensetzungen, die pankreatisches Hormon produzierende Zellen enthalten und insbesondere mit dem genannten Verfahren gebildet werden, Anwendungen derartiger Zellen und Zellzusammensetzungen, insbesondere pharmazeutische Anwendungen dieser Zellen und Zellzusammensetzungen.

Es ist bekannt, Stammzellen in Insulin oder andere pankreatische Hormone produzierende Zellen zu differenzieren (siehe DE 102 90 025 T1, WO 02/059278). Die herkömmlichen Differenzierungstechniken besitzen wesentliche Nachteile sowohl in Bezug auf die Vorläuferzellen für die Zelldifferenzierung als auch in Bezug auf die komplexen Verfahrensbedingungen, die zur Zelldifferenzierung eingehalten werden müssen. Die herkömmlichen Verfahren basieren in der Praxis auf der Differenzierung der Zellen aus Aggregaten embryonaler Stammzellen (sog. Embryoide). Die massenhafte Verwendbarkeit von Embryoiden zur Zelldifferenzierung ist jedoch aus einer Reihe von Gründen und insbesondere ethischen Erwägungen ausgeschlossen. In DE 102 90 025 T1 wird zwar die Differenzierung von adulten Stammzellen vorgeschlagen. Die mit den herkömmlichen Verfahren gewonnenen adulten Stammzellen liefern jedoch nur beschränkt die gewünschten Differenzierungsergebnisse.

Die Aufgabe der Erfindung ist es, verbesserte Verfahren zur Differenzierung von Stammzellen in Zellen, die mindestens ein pankreatisches Hormon produzieren, bereitzustellen, mit denen die Beschränkungen in Bezug auf die Verwendung embryonaler Stammzellen vermieden werden und die dennoch für eine massenhafte Anwendung unter einfachen Verfahrensbedingungen geeignet sind. Die Aufgabe der Erfindung besteht insbesondere in der Schaffung eines verbesserten Verfahrens zur Erzeugung von pankreatisches Hormon produzierenden Zellen für die Transplantation in Patienten, die an pankreatischen Erkrankungen insbesondere an Diabetes leiden.

Diese Aufgabe wird durch ein Verfahren mit den Merkmalen von Anspruch 1, eine Zelle mit den Merkmalen von Anspruch 22 und eine Zellzusammensetzung mit den Merkmalen von Anspruch 24 gelöst. Vorteilhafte Ausführungsformen und Anwendungen ergeben sich aus den abhängigen Ansprüchen.

Verfahrensbezogen wird die o.g. Aufgabe durch eine Kultivierung und Differenzierung von nicht-embryonalen Stammzellen gelöst, die aus dem Gewebe einer differenzierten exokrinen Drüse eines Organismus gewonnen wurden. Ein besonderer Vorteil dieses Verfahrens besteht in der Bildung von Zellen, die pankreatisches Hormon produzieren (im folgenden: hormonproduzierende Zellen), ohne die bisher verwendete Differenzierung aus embryonalen Stammzellen. Die Erfinder haben festgestellt, dass die aus exokrinem Drüsengewebe isolierten adulten Stammzellen pluripotent sind und eine hohe Teilungsfähigkeit und ein starkes Wachstum zeigen. Damit wird eine effektive Quelle für differenzierungsfähige Zellen bereitgestellt, aus denen die hormonproduzierenden Zellen massenhaft gebildet werden können.

Das erfindungsgemäß verwendete exokrine Drüsengewebe kann von einem erwachsenen Organismus, juvenilen Organismus oder nicht-humanen fötalen Organismus, vorzugsweise einem postnatalen Organismus, stammen. Der Begriff "adult", wie in der vorliegenden Anmeldung verwendet, bezieht sich somit auf das Entwicklungsstadium des Ausgangsgewebes und nicht auf dasjenige des Donororganismus, aus dem das Gewebe stammt. "Adulte" Stammzellen sind nicht-embryonale Stammzellen.

Vorzugsweise wird das exokrine Drüsengewebe aus einer Speicheldrüse, Tränendrüse, Talgdrüse, Schweißdrüse, aus Drüsen des Genitaltrakts, einschließlich Prostata, oder aus gastrointestinalem Gewebe, einschließlich Pankreas, oder sekretorischem Gewebe der Leber isoliert. In einer stark bevorzugten Ausführungsform handelt es sich dabei um acinäres Gewebe. Ganz besonders bevorzugt stammt das acinäre Gewebe aus dem Pankreas, der Ohrspeicheldrüse oder Unterkieferspeicheldrüse.

Ein weiterer wichtiger Vorteil des erfindungsgemäßen Verfahrens besteht darin, dass die Stammzellen effektiv aus lebenden Donororganismen, z. B. aus Speicheldrüsen von Säugetieren oder aus humanen Speicheldrüsen gewonnen werden können, ohne dass der Donororganismus entscheidend beeinträchtigt wird. Dies ist sowohl unter ethischen Gesichtspunkten als auch mit Blick auf die Möglichkeit der weiteren Beobachtung des Donororganismus hinsichtlich eventueller Krankheiten von besonderem Vorteil.

Gemäß einer ersten Ausführungsform der Erfindung werden die primär aus dem Organismus isolierten Stammzellen als Quelle für die weitere Kultivierung und Differenzierung hin zu hormonproduzierenden Zellen verwendet. Diese Variante besitzt den Vorteil einer besonders einfachen Verfahrensführung. Die gewünschten differenzierten Zellen können direkt aus einer Primärkultur gewonnen werden. Alternativ ist gemäß einer abgewandelten Ausführungsform der Erfindung vorgesehen, dass zunächst eine Aggregation der aus dem Organismus isolierten Stammzellen zu so genannten organoiden Körpern erfolgt. Diese Variante besitzt den Vorteil, dass mit den organoiden Körpern ein effektives Reservoir für größere Mengen differenzierter Zellen geschaffen wird. Die Erfinder haben festgestellt, dass die aus dem exokrinen Drüsengewebe isolierten Stammzellen organoide Körper bilden, die bei Nährstoffversorgung ein starkes Wachstum zu Gewebekörpern mit Durchmessern bis zu einigen Millimetern oder darüber zeigen.

Gemäß einer ersten Variante der Verwendung organoider Körper als Quelle für die gewünschten hormonproduzierenden Zellen ist erfindungsgemäß vorgesehen, dass die Differenzierung der Stammzellen in den organoiden Körpern erfolgt und anschließend die differenzierten hormonproduzierenden Zellen aus den organoiden Körpern entnommen werden. Gemäß einer zweiten Variante werden sekundär aus den organoiden Körpern Stammzellen im undifferenzierten Zustand isoliert, kultiviert und zu den gewünschten hormonproduzierenden Zellen differenziert. Diese Ausführungsform besitzt den Vorteil einer besonders einfachen Verfahrensführung, da die organoiden Körper in Adhäsionskultur ein spontanes Auswandern und Auswachsen von Stammzellen oder differenzierten Zellen zeigen, die dann für die weitere Kultivierung und Differenzierung zur Verfügung stehen.

Grundsätzlich kann das erfindungsgemäße Verfahren ausgeführt werden, indem hormonproduzierende Zellen, die sich spontan aus den primär oder sekundär isolierten Stammzellen oder aus den organoiden Körpern gebildet haben, selektiert und weiter vermehrt werden. Gemäß einer bevorzugten Ausführungsform der Erfindung ist bei der Differenzierung der hormonproduzierenden Zellen eine Stimulation der Zellkultur vorgesehen. Die Stimulation besitzt den Vorteil einer erhöhten Effektivität und Geschwindigkeit der Bildung der gewünschten hormonproduzierenden Zellen. Gemäß einer ersten Variante ist nach der Differenzierung der Stammzellen zu den hormonproduzierenden Zellen deren stimulierte Vermehrung in einem Kultivierungsmedium vorgesehen. Gemäß einer zweiten Variante ist die Stimulation auf eine stimulierte Differenzierung der Stammzellen hin zu den gewünschten hormonproduzierenden Zellen vorgesehen.

Erfindungsgemäß kann die Stimulation eine oder mehrere der folgenden Stimulationsbehandlungen umfassen, die gleichzeitig oder aufeinander folgend durchgeführt werden können. Es kann eine Behandlung mit Überständen einer Primärkultur des endokrinen Pankreas oder von Zelllinien des endokrinen Pankreas, eine Co-Kultivierung mit differenzierten Zellen des endokrinen Pankreas oder mit davon abgeleiteten Zelllinien, eine Behandlung (Prägung) mit immobilisierten oder gelösten, in der flüssigen Phase bereitgestellten molekularen Differenzierungsfaktoren oder eine Genaktivierung in der Stammzelle vorgesehen sein. Des weiteren kann eine Stimulation durch den Zusatz von bereits differenzierten hormonproduzierenden Zellen und/oder anderen Substanzen, zum Beispiel Hormone, oder Zelltypen, die die Differenzierung beeinflussen, erzielt werden.

Wenn die Prägung mit immobilisierten Wachstumsfaktoren erfolgt, so werden vorzugsweise Differenzierungsfaktoren verwendet, die auf einem beweglichen, relativ zu den Stammzellen positionierbaren Träger fixiert sind. Vorteilhafterweise kann damit eine gezielte Differenzierung einzelner Stammzellen oder bestimmter Stammzellgruppen erreicht werden. Der Träger ist beispielsweise ein synthetisches Substrat, das Vorteile für eine gezielte Gestaltung mit den Differenzierungsfaktoren besitzt, oder eine biologische Zelle, auf deren Zellmembran die Differenzierungsfaktoren angeordnet sind.

Wenn gemäß einer weiteren bevorzugten Ausführungsform der Erfindung eine Identifizierung und Selektion der differenzierten Zellen aus der Zellkultur vorgesehen ist, können sich Vorteile für die weitere Verwendung der gebildeten pankreatisches hormonproduzierenden Zellen ergeben. Es kann insbesondere eine Zellzusammensetzung bereitgestellt werden, die vollständig oder zum größten Teil aus hormonproduzierenden Zellen besteht. Wenn die Selektion mit Sortierverfahren erfolgt, die an sich bekannt sind, wie z. B. mit einem präparativen Zellsorter-Verfahren oder eine Sortierung in einem fluidischen Mikrosystem, können sich Vorteile für die Kompatibilität mit herkömmlichen zellbiologischen Prozeduren ergeben.

Ein weiterer Vorteil der Identifizierung und Selektion besteht darin, dass Zellen, die nicht als hormonproduzierende Zellen identifiziert sind und entsprechend nicht aus der bearbeiteten Kultur selektiert werden, einer weiteren Kultivierung und Differenzierung unterzogen werden können. Damit kann vorteilhafterweise die Ausbeute des erfindungsgemäßen Verfahrens gesteigert werden.

Gemäß bevorzugten weiteren Varianten der Erfindung werden zur Bildung der hormonproduzierenden Zellen Stammzellen aus Gewebe von sekretorischen Drüsen oder Drüsen des gastro-intestinalen Traktes des Organismus gewonnen. Die Stammzellen werden insbesondere aus Gewebe isoliert, das aus acinärem Gewebe besteht oder acinäres Gewebe enthält. Bei der Gewinnung aus der Pankreas können sich Vorteile für die Verwendung weiterer Gewebeteile der Pankreas für die genannte Stimulation ergeben. Bei der Gewinnung aus der Speicheldrüse können sich Vorteile für die schonende Behandlung des Donororganismus ergeben.

Bevorzugte Donororganismen sind Wirbeltiere, insbesondere Säuger, wie z. B. der Mensch. Bei der Verwendung humaner Stammzellen erfolgt die Isolation der Stammzellen aus nicht-embryonalen Zuständen, also aus differenziertem Gewebe in der juvenilen Phase oder der adulten Phase. Bei nichtmenschlichen Donororganismen kann grundsätzlich auch auf differenziertes Gewebe im fötalen Zustand zurückgegriffen werden.

Die erfindungsgemäß hergestellten hormonproduzierenden Zellen werden vorzugsweise therapeutisch verwendet. Gemäß einer ersten Variante kann eine Humanbehandlung mit hormonproduzierenden Zellen vorgesehen sein, die aus tierischen Organismen gewonnen wurden. Des Weiteren sind Behandlungen eines Menschen mit hormonproduzierenden Zellen möglich, die aus einem anderen Menschen gewonnen wurden. Besonders bevorzugt ist die autologe Behandlung eines Menschen mit hormonproduzierenden Zellen, die von eigenen, juvenilen oder adulten Stammzellen gewonnen wurden. Die Behandlung kann pankreatische Erkrankungen, Stoffwechselsyndrome oder Stoffwechselerkrankungen, insbesondere die Diabetes, die Hyperglykämie oder eine beeinträchtigte Glukosetoleranz betreffen. Besonders bevorzugt wird als pankreatisches Hormon Insulin produziert.

Einen unabhängigen Gegenstand der Erfindung stellt eine isolierte, ein pankreatisches Hormon produzierende Zelle dar, die aus einer Stammzelle differenziert wurde, welche aus differenziertem endokrinen Drüsengewebe eines Organismus stammt. Eine derartig isolierte Zelle wird vorzugsweise mit dem erfindungsgemäßen Verfahren gewonnen.

Ein weiterer unabhängiger Gegenstand der Erfindung ist eine Zellzusammensetzung, die eine Vielzahl derartiger pankreatisches Hormon produzierender Zellen enthält. Die Zellzusammensetzung wird vorzugsweise durch das erfindungsgemäße Verfahren gewonnen. Gemäß einer bevorzugten Ausführungsform der Erfindung kann die Zellzusammensetzung weitere Zellen oder Materialien enthalten, die bspw. eine Matrix bilden. Entsprechend ist ein weiterer Gegenstand der Erfindung eine künstliche Langerhans'sche Insel.

Gemäß einer bevorzugten Ausführungsform der Erfindung umfasst die Zellzusammensetzung eine Umhüllung oder eine 3-dimensionale Matrix, in der die hormonproduzierenden Zellen und weitere Zelltypen angeordnet sind. Die Umhüllung oder 3-dimensionale Matrix besteht zum Beispiel aus Alginat, Kollagen, implantierbaren Materialien, Polymeren (Biopolymere oder synthetische Polymere). Es wird ein semipermeables Material verwendet, das das Hormon (zum Beispiel Insulin) nach außen durchlässt, nicht jedoch die Zellen. Die weiteren Zelltypen umfassen zum Beispiel Stammzellen und/oder Nachbarzellen von Langerhans'schen Inseln im pankreatischen Gewebe. Die Bildung der Umhüllung (Kapsel) wird bevorzugt, da mit dieser besonders effektiv insbesondere die hormonproduzierenden Zellen immobilisiert und ggf. an einem weiteren Wachstum gehindert werden. Das hier beschriebene Kapsel-Zell-Komposit oder Matrix-Zell-Komposit besitzt einen Durchmesser von einigen 100 µm bis einige mm.

Ein weiterer unabhängiger Gegenstand der Erfindung ist eine pharmazeutische Zusammensetzung mit mindestens einer erfindungsgemäß hergestellten hormonproduzierenden Zelle oder Zellzusammensetzung und einer pharmazeutischen Trägersubstanz. Als pharmazeutische insbesondere flüssige Trägersubstanz kann jedes Material verwendet werden, das an sich aus der Pharmazie für diese Funktion bekannt ist.

Weitere Vorteile und Einzelheiten der Erfindung werden im Folgenden unter Bezug auf die beigefügten Zeichnungen beschrieben. Es zeigen:
Figur 1: ein Flussdiagramm eines Differenzierungsverfahrens gemäß einer Ausführungsform der Erfindung,
Figur 2: ein Flussdiagramm zur Illustration verschiedener Varianten der Bereitstellung von Stammzellen,
Figur 3: ein Flussdiagramm zur Illustration verschiedener Varianten der Aggregation von Stammzellen zu organoiden Körpern,
Figur 4: eine Prozedur zur Herstellung organoider Körper,
Figur 5: eine Illustration der Kultivierung von organoiden Körpern,
Figuren 6 und 7: schematische Illustrationen der Prägung von Stammzellen durch molekulare Signalfaktoren, und
Figur 8: eine Illustration des Nachweises der Insulinproduktion erfindungsgemäß differenzierter Zellen.

### (I) Isolierung und Aggregation von Stammzellen aus einer differenzierten exokrinen Drüse eines lebenden Organismus zu organoiden Körpern

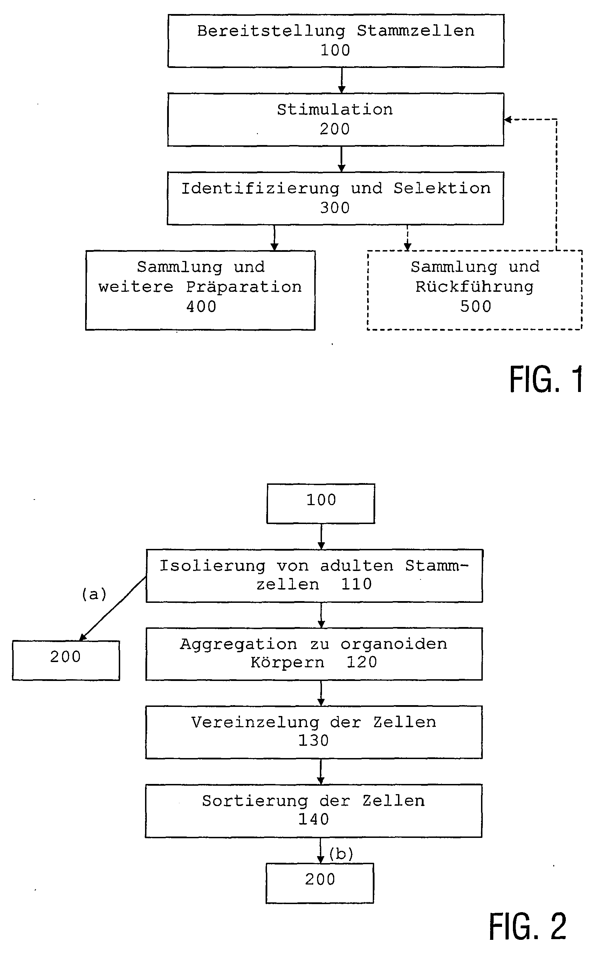

Die erfindungsgemäße Kultivierung und Differenzierung von Stammzellen, die aus differenziertem exokrinen Drüsengewebe eines Organismus gewonnen wurden, umfasst die in Figur 1 illustrierten Schritte. Zunächst erfolgt die Isolierung von Stammzellen aus dem Organismus (Schritt 100), um eine Quelle für die Stimulation und Differenzierung hin zu den hormonproduzierenden Zellen zu bilden. Einzelheiten der Isolierung und verschiedene Varianten von Quellen für die weiteren Verfahrensschritte sind unten erläutert. Anschließend folgt im Rahmen der Differenzierung die Stimulation (Schritt 200) einer als Quelle verwendeten Zellkultur. Nach der Differenzierung erfolgt bei Schritt 300 eine Identifizierung der hormonproduzierenden Zellen und eine entsprechende Selektion. Die ausgewählten hormonproduzierenden Zellen werden gesammelt und ggf. weiter für eine z. B. pharmazeutische Anwendung präpariert (Schritt 400). Des Weiteren kann eine Rückführung der nicht differenzierten Zellen in die Ausgangskultur vorgesehen sein (Schritt 500), um erneut der Stimulation und Differenzierung ausgesetzt zu werden.

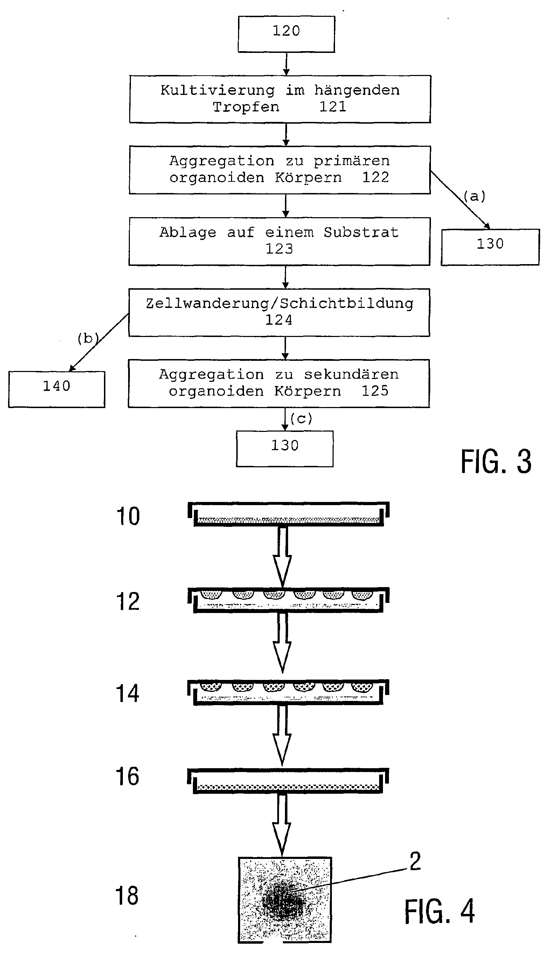

Verschiedene Varianten der Bereitstellung von undifferenzierten Stammzellen für die weitere Stimulation sind in den Figuren 2 und 3 illustriert. Gemäß Figur 2 erfolgt zunächst die Isolierung von Stammzellen aus dem Donororganismus (Schritt 110, Beispiele siehe unten). Ausgehend von diesen primär isolierten Stammzellen kann gemäß einer ersten Variante (a) direkt zur Stimulation (Schritt 200) übergegangen werden. Alternativ erfolgt bei Schritt 120 zunächst eine Aggregation der Stammzellen zu organoiden Körpern. Einzelheiten des Aggregationsschrittes sind in den Figuren 3 und 4 zusammengestellt. Aus den organoiden Körpern können bei Schritt 130 Stammzellen oder bereits differenzierte Zellen vereinzelt und/oder sortiert (Schritt 140) werden, wobei dann gemäß Variante (b) mit der weiteren Stimulation der Differenzierung oder der Vermehrung der bereits differenzierten Zellen fortgefahren werden kann.

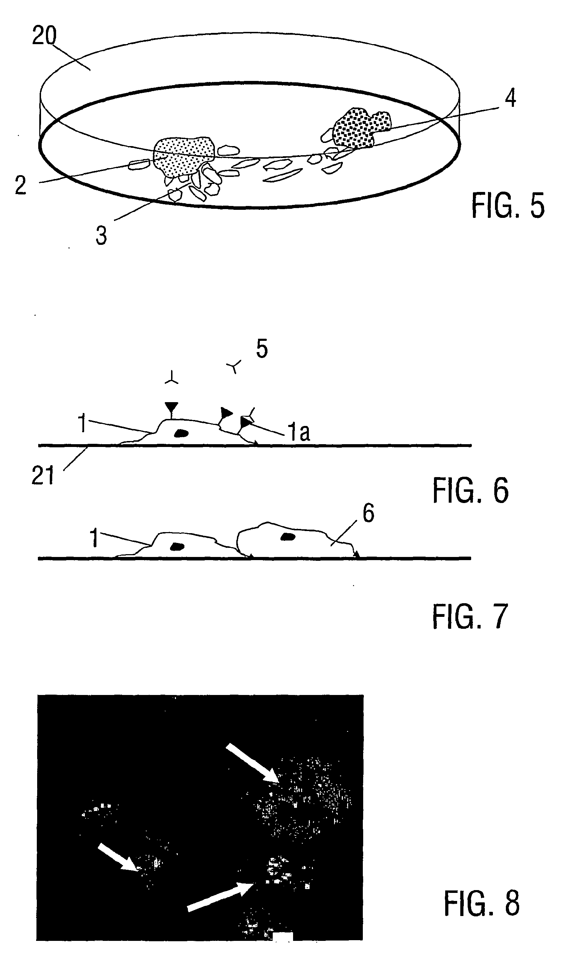

Gemäß Figur 3 umfasst die Aggregation von Stammzellen zu organoiden Körpern gemäß Schritt 120 die folgenden Teilschritte. Zunächst erfolgt die Kultivierung der Stammzellen in hängenden Tropfen (Schritt 121, Beispiel siehe unten). Alternativ kann die Kultivierung in einer Schüttelkultur oder in einem schwebenden Zustand ohne Kontakt mit festen Grenzflächen in elektromagnetischen Feldkäfigen erfolgen. Im hängenden Tropfen oder der entsprechenden dreidimensionalen Suspensionskultur erfolgt die Aggregation zu primären organoiden Körpern (Schritt 122). Diese können als Quelle für die Vereinzelung von Zellen verwendet werden. Gemäß Variante (a) in Figur 4 erfolgt hier der Sprung zu Schritt 130 in Figur 2. Alternativ werden die primären organoiden Körper zunächst auf einem Substrat in einem Kultivierungsmedium für eine Adhäsionskultur abgelegt (Schritt 123). Auf dem Substrat erfolgt eine Zellwanderung und eine Schichtbildung (Schritt 124, siehe Figur 5). Im Ergebnis liegen nach Schritt 124 vereinzelte Stammzellen oder differenzierte Zellen vor, so dass gemäß Variante (b) in Figur 3 unmittelbar der Sprung zu Schritt 140 in Figur 2 erfolgen kann.

Eine Besonderheit der organoiden Körper in Adhäsionskultur besteht in der Bildung von sekundären organoiden Körpern aus der in der Adhäsionskultur gebildeten Zellschicht (Schritt 125). Aus den sekundären organoiden Körpern oder entsprechend abgeleiteten organoiden Körpern können wiederum gemäß Variante (c) in Figur 3 Zellen vereinzelt werden (Sprung zu Schritt 130 in Figur 2).

Die Vereinzelung der differenzierten Zellen aus einer Adhäsionskultur erfolgt nach an sich bekannten Verfahren, z.B. unter Verwendung von Markersubstanzen, die für die pankreatischen Hormone charakteristisch sind. Es erfolgt beispielsweise ein Trypsinieren der in Adhäsionskultur differenzierten Zellen und deren Überführung in eine Suspension. In dieser erfolgt eine Identifizierung zum Beispiel unter Verwendung einer an sich bekannten Zellsortiereinrichtung mit einem fluidischen Mikrosystem, mit einem präparativen Zellsorter-Verfahren oder mit magnetischen Beads, an die Antikörper angekoppelt sind, die nur mit den hormonproduzierenden Zellen in der Suspension ankoppeln. Beim präparativen Zellsorter-Verfahren wird gezielt nach Oberflächenmarkern gesucht und die hormonproduzierenden Zellen mit spezifischen Oberflächenmarkern selektiert.

Die Identifizierung der gesuchten Zellen kann auch in der Zellkultur zum Beispiel auf der Grundlage morphologischer Unterscheidungsmerkmale erfolgen, durch die sich die hormonproduzierenden Zellen von den undifferenzierten Zellen oder anderen Zelltypen unterscheiden. Morphologische Unterscheidungsmerkmale beziehen sich zum Beispiel auf die Geometrie der Zelle oder die Anordnung des Zellkerns oder granulärer Bestandteile in der Zelle.

Die Identifizierung und Selektion kann ferner durch eine zellelektrophoretische Untersuchung oder eine Untersuchung einer anderen, spezifischen nativen Eigenschaft, wie zum Beispiel der Oberflächenbeweglichkeit, der hormonproduzierenden Zellen erfolgen.

Es können ferner mehrstufige oder aus den genannten Techniken kombinierte Identifizierungen und Selektionen vorgesehen sein.

Entsprechend dem in Figur 4 dargestellten Schema wird zur Gewinnung der Zellen acinäres Gewebe - bevorzugt einer Speicheldrüse oder der Bauchspeicheldrüse (Pankreas) - mechanisch und enzymatisch zerkleinert in Kultur genommen (Schritt 10 in Figur 2). Entgegen den Angaben von Bachem et al., Gastroenterol. 115:421-432 (1998), und Grosfils et al., Res. Comm. Chem. Pathol. Pharmacol. 79:99-115 (1993), werden keine Gewebeblöcke kultiviert, aus denen Zellen auswachsen sollen, sondern unter der Bedingung, dass die Zellverbände der Acini weitestgehend intakt bleiben, das Gewebe stärker zerkleinert.

Über mehrere Wochen werden diese Zellen und Zellverbände in Kulturgefäßen kultiviert. Alle 2 bis 3 Tage wird das Medium gewechselt, wobei alle differenzierten Zellen entfernt werden. Bei den in Kultur persistierenden Zellen handelt es sich um undifferenzierte Zellen mit uneingeschränkter Teilungsfähigkeit.

Ähnliche Zellen sind unter gleichen Bedingungen aus dem Pankreas isoliert und beschrieben und als eine Art Myofibroblasten bzw. pankreatische Sternzellen bezeichnet worden (Bachem et al., 1998). Im Gegensatz zu den Zellen der vorliegenden Erfindung konnte eine uneingeschränkte Teilungsfähigkeit jedoch nicht beobachtet werden. Weiterhin konnten diese Zellen auch nicht unbegrenzt passagiert werden, ohne an Vitalität zu verlieren.

In einem zweiten Schritt (12) werden ungefähr 400 bis 800 Zellen in je 20 µl Medium in hängenden Tropfen kultiviert. Dazu werden die Tropfen auf Deckel von bakteriologischen Petrischalen gegeben, umgedreht und über die mit Medium gefüllte Petrischale gelegt, so dass die Tropfen nach unten hängen.

Durch diese Art der Kultivierung bilden sich innerhalb von 48h die als organoide Körper bezeichneten Zellaggregate (14), die für ungefähr 6 Tage in eine Suspensionskultur umgesetzt werden (16). Die Teilansicht (18) aus Figur 2 zeigt eine mikroskopische Aufnahme eines solchen organoiden Körpers 2.

Die Bildung der o. g. sekundären organoiden Körper ist in Figur 5 illustriert. Die primären organoiden Körper 2 bilden auf dem Substrat der Adhäsionskultur, wie z. B. auf dem Boden der Kulturschale 20, durch ein Wandern oder Wachsen von Zellen 3 zunächst eine Monoschicht, aus der dann die sekundären organoiden Körper 4 herauswachsen. Mit der Kultivierung der primären organoiden Körper 2 zu sekundären organoiden Körpern 4 wird eine weitere Vervielfältigung des Zellmaterials geschaffen. Aus jedem der primären oder sekundären organoiden Körper 2, 4 können die hormonproduzierenden Zellen gewonnen werden.

### Weitere Ausführungsbeispiele für die Isolierung und Aggregation von Stammzellen

In den folgenden, nicht-beschränkenden Beispielen wird die Isolierung und Aggregation von Stammzellen näher erläutert.

Die allgemeinen Arbeitsanweisungen, wie sie für Verfahren zur Kultivierung von biologischen Zellen und insbesondere von Säugetierzellen gebräuchlich sind, sind zu beachten. Eine sterile Umgebung, in der das Verfahren durchgeführt werden soll, ist - auch wenn hierzu keine weitere Beschreibung erfolgt - in jedem Fall einzuhalten. Folgende Puffer und Medien wurden verwendet:

| | |
|---|---|
| HEPES-Stammlösung (pH 7,6) | 2,3 83 g HEPES auf 100 ml A. *bidest.* |
| HEPES-Eagle-Medium (pH 7,4) | 90 ml Modified Eagle Medium (MEM) |
| | 10 ml HEPES-Stammlösung |
| Isolationsmedium (pH 7,4) | 32 ml HEPES-Eagle-Medium |
| | 8 ml 5% BSA in A. bidest. |
| | 300 µl 0,1 M CaC12 |
| | 100 µl Trasylol (200.000 KIE) |
| Digestionsmedium (pH 7,4) | 20 ml Isolationsmedium |
| | 4 ml Kollagenase (Kollagenase NB 8 von Serva) |
| Inkubationsmedium | Dulbecco's Modified Eagle Medium (DMEM) |
| Nährmedium | Dulbecco's Modified Eagle Medium (DMEM) |
| | DMEM + 4500 mg/l Glucose |
| | + L-Glutamin |
| | - Pyruvat |
| | + 20 % FKS (inaktiviert) + 1 ml/100 ml Pen/Strep |
| | (10000 E/10000 µg/ml) |
| | oder |
| | DMEM + 10 % Eigenplasma + 1 ml/100 ml |
| | Pen/Strep, |
| | vor Gebrauch auf 37°C erwärmen |
| Differenzierungsmedium (I) | 380 ml DMEM |
| | 95 ml 30 min bei 54 °C inaktiviertes FKS |
| | 5 ml Glutamin (GIBCO BRL) |
| | 5 ml (3,5 µl β-Mercaptoethanol auf 5 ml PBS) |
| | 5 ml Non-essential amino acids (GIBCO BRL) |
| | 5 ml Penicillin/Streptomycin (GIBCO BRL) |
| | (10000 E/10000 µg/ml) |

Statt fötalem Kalbserum (FKS) im Nährmedium und Differenzierungsmedium kann gegebenenfalls auch Eigenplasma oder, weniger bevorzugt, Eigenserum des Gewebedonors verwendet werden. Dies ist insbesondere dann von Bedeutung, wenn der Gewebedonor mit dem späteren Empfänger der Stammzellen oder davon abgeleiteten differenzierten Zellen identisch ist. Eine solche autologe Behandlung ist zur Verhinderung einer eventuellen Abstoßungsreaktion bevorzugt.

Das Nährmedium kann als Basismedium statt des verwendeten DMEM-Mediums auch ein anderes für die Kultivierung von eukaryotischen Zellen, insbesondere Säugerzellen, bekanntes, geeignetes Basismedium enthalten, in dem die differenzierten Zellen absterben und sich die gewünschten Stammzellen vermehren. Auch Isolationsmedium, Inkubationsmedium und Differenzierungsmedium können ein anderes übliches und geeignetes Basismedium enthalten.

Weitere Differenzierungsmedien, die zur erfindungsgemäß vorgesehenen Stimulation verwendet werden können bestehen in den Überständen von Primärkulturen oder abgeleiteten Zelllinien des endokrinen Pankreas oder in Zellsuspensionen mit differenzierten Zellen oder abgeleiteten Zellen des endokrinen Pankreas. Es können insbesondere Zellen oder Zellgruppen verwendet werden, die im endokrinen Gewebe der Pankreas in der Umgebung der Langerhans'schen Inseln angeordnet sind.

Die folgenden Beispiele 1 und 2 beschreiben detailliert zwei Arbeitsprotokolle zur Isolierung und Kultivierung adulter pluripotenter Stammzellen aus acinärem Gewebe des Pankreas. Die Beispiele 1 und 2 beziehen sich auf die Isolierung von Stammzellen aus Ratten. Die Isolierung aus anderen Säugetieren, z.B. Schweinen erfolgt analog. Beispiel 3 beschreibt ein entsprechendes Protokoll für die Isolierung aus acinärem Gewebe der Speicheldrüse.

### BEISPIEL 1

### 1. Präparation des Gewebes und Isolierung der Zellen

In den *Ductus pancreaticus* von 2-3 Jahre alten Ratten werden mittels einer Spritze und einer stumpfen Kanüle bei der Ratte 10 ml Digestionsmedium langsam und luftblasenfrei injiziert. Das gesamte Pankreas wird dadurch aufgeblasen und kann so besser herauspräpariert werden. Das Pankreas wird dann in ein Becherglas überführt und weitere 5 ml Digestionsmedium dazugegeben. Nachdem man das Fettgewebe und Lymphknoten entfernt hat, wird das Gewebe im Becherglas mit einer feinen Schere sehr fein zerkleinert, oben schwimmendes Fettgewebe abgesaugt und die Suspension wird abschließend 1 min mit Carbogen begast (wenn nötig wiederholen) und für 20 min bei 37 °C mit Alufolie bedeckt in einem Schüttler bei 200 Zyklen/min inkubiert. Danach saugt man das Medium vorsichtig ab, zerkleinert erneut mit einer Schere das Gewebe und wäscht die Gewebestücke zweimal mit je 10 ml Isolationsmedium und gibt wieder 5 ml Digestionsmedium zum Gewebe hinzu.

Nach erneutem Begasen mit Carbogen für etwa 1 min und Inkubation für 15 min bei 37 °C in einem Schüttler bei 200 Zyklen/min werden die Gewebestücke durch sukzessives Aufziehen in jeweils einer 10 ml, 5ml, 2 ml und 1 ml Glaspipette zerkleinert und durch einlagiges Filtergewebe gepresst. Die so vereinzelten Zellen werden nun fünfmal in Inkubationsmedium gewaschen (37 °C), mit Carbogen begast und jedes Mal 5 min bei 90 g zentrifugiert. Das zuletzt erhaltene Pellet wird in Inkubationsmedium resuspendiert, begast und auf Gewebekulturschalen verteilt.

### 2. Kultivierung der Zellen

Die Gewebekulturschalen mit den isolierten Zellen werden im Brutschrank bei 37°C und 5 % CO₂ kultiviert. Alle 2-3 Tage wird das Medium gewechselt. Dabei werden alle differenzierten Zellen entfernt.

Am siebenten Tag in Kultur werden die Zellen mit einer Lösung bestehend aus 2 ml PBS, 1 ml Trypsin und 2 ml Inkubationsmedium passagiert. Dabei lösen sich die Zellen vom Boden der Kulturschale. Die Zellsuspension wird 5 Minuten zentrifugiert, der Überstand abgesaugt und die Zellen in 2 ml Inkubationsmedium resuspendiert, auf eine mittlere Zellkulturflasche überführt und 10 ml Inkubationsmedium dazugegeben. Der Medienwechsel erfolgt alle drei Tage.

Am vierzehnten Tag in Kultur werden die Zellen erneut, aber diesmal mit 6 ml PBS, 3 ml Trypsin und 6 ml Inkubationsmedium, passagiert. Die Zellsuspension wird 5 Minuten zentrifugiert, der Überstand abgesaugt und die Zellen in 6 ml Inkubationsmedium resuspendiert, auf 3 mittlere Zellkulturflaschen überführt und jeweils 10 ml Inkubationsmedium dazugegeben.

Die Zellen werden weiter kultiviert und so oft passagiert und ausgesät, bis die Zellen einen semikonfluenten bis konfluenten Zustand erreichen.

### BEISPIEL 2

Pankreas-Acini wurden von männlichen Sprague-Dawley-Ratten (20-300 g) erhalten, die narkotisiert (CO₂) und über die dorsale Aorta ausgeblutet worden waren. Eine Kanüle wurde trasduodenal in den Pankreasgang eingeführt und dem Pankreas wurde von hinten 10 ml Digestionsmedium, enthaltend HEPES-Eagle-Medium (pH 7,4), 0,1 mM HEPES-Puffer (pH, 7,6), 70% (Vol./Vol.) modifiziertes Eagle-Medium, 0,5 % (Vol./Vol.) Trasylol (Bayer AG, Leverkusen, Deutschland), 1 % (Gew./Vol.) Rinderserumalbumin), 2,4 mM CaCl₂ und Kollagenase (0,63 P/mg, Serva, Heidelberg, Deutschland) injiziert.

Vor der Entfernung wurde der Pankreas von anhaftendem Festgewebe, Lymphknoten und Blutgefäßen teilweise befreit. Dann wurde gesundes Pankreasgewebe in Digestionsmedium abgeholt (bei 20 °C, geringerer Stoffwechsel), das Pankreasgewebe mit einer Schere sehr fein zerkleinert, oben schwimmendes Fettgewebe abgesaugt und die Gewebesuspension mit Carbogen (Messer, Krefeld, Deutschland) begast, ohne dass die Düse in das Medium mit den Zellen gelangt (Verringerung von mechanischem Streß) und damit auf pH 7,4 eingestellt. Danach wurde die Suspension in einem 25-ml-Erlenmeyerkolben (mit Alufolie bedeckt) unter konstantem Schütteln (150-200 Zyklen pro Minute) bei 37 °C in 10 ml Digestionsmedium inkubiert. Nach 15-20 Minuten wurde das oben schwimmende Fett und das Medium abgesaugt und das Gewebe wurde erneut zerkleinert und mit Medium ohne Kollagenase gespült (Vorgang mindestens zweimal wiederholen, vorzugsweise solange bis Zellfraktion transparent), worauf Digestionsmedium zugegeben und erneut etwa 1 Minute lang mit Carbogen begast wurde. Es folgte wiederum eine Digestion mit Kollagenase für 15 Minuten bei 37°C im Schüttler unter Verwendung desselben Puffers. Nach der Digestion wurden die Acini durch sukzessives Hochziehen und Ausstossen durch 10 ml-, 5 ml und 2 ml-Glaspipetten mit engen Öffnungen dissoziiert und durch ein einlagiges Nylonsieb (Polymon PES-200/45, Angst & Pfister AG, Zürich, Schweiz) mit einer Maschengröße von etwa 250 µm filtriert. Die Acini wurden zentrifugiert (bei 37°C und 600-800 UpM in einer Beckman-GPR-Zentrifuge, entspricht etwa 90 g) und weiter gereinigt durch Waschen in Inkubationsmedium, enthaltend 24,5 mM HEPES (pH 7,5), 96 mM NaCl, 6 mM KCl, 1 mM MgCl₂, 2,5 mM NaH₂PO₄, 0, mM CaCl₂, 11,5 mM Glucose, 5 mM Natriumpyruvat, 5 mM Natriumglutamat, 5 mM Natriumfumarat, 1% (Vol./Vol.) modifiziertes Eagle-Medium, 1 % (Gew./Vol.) Rinderserumalbumin, mit Carbogen äquilibriert und auf pH 7,4 eingestellt. Die Waschprozedur (Zentrifugation, Absaugen, Resuspension) wurde fünfmal wiederholt. Soweit nicht anders angegeben, wird bei der obigen Isolierung bei etwa 20 °C gearbeitet.

Die Acini wurden in Inkubationsmedium resuspendiert und bei 37°C in einer angefeuchten Atmosphäre mit 5% CO₂ kultiviert. Das acinäre Gewebe starb dabei schnell (innerhalb von zwei Tagen) ab und die sterbenden differenzierten Zellen lösten sich dabei von den benachbarten Zellen, ohne diese zu schädigen (schonende Isolierung), die nicht absterbenden Stammzellen sanken zu Boden und hefteten sich an. Die differenzierten Acinizellen sind dazu nicht in der Lage. Das Inkubationsmedium wurde am zweiten oder dritten Tag nach dem Aussäen erstmals gewechselt, wobei ein Großteil der frei schwimmenden Acini und acinären Zellen entfernt wurde. Zu diesem Zeitpunkt hatten sich die ersten Stammzellen bzw. deren Vorläufer am Boden festgesetzt und begannen sich zu teilen. Der Mediumwechsel wurde danach an jedem dritten Tag wiederholt und differenzierte acinäre Pankreaszellen wurden bei jedem Mediumwechsel entfernt.

Am siebenten Tag in Kultur wurden die Zellen mit einer Lösung bestehend aus 2 ml PBS, 1 ml Trypsin (+ 0,05 % EDTA) und 2 ml Inkubationsmedium passagiert. Dabei lösen sich die Zellen vom Boden der Kulturschale. Die Zellsuspension wurde 5 Minuten bei etwa 1000 UpM (Beckmann GPR-Zentrifuge) zentrifugiert, der Überstand abgesaugt und die Zellen in 2 ml Inkubationsmedium resuspendiert, auf eine mittlere Zellkulturflasche überführt und 10 ml Inkubationsmedium dazugegeben.

Am vierzehnten Tag in Kultur wurden die Zellen erneut, aber diesmal mit 6 ml PBS, 3 ml Trypsin/EDTA und 6 ml Inkubationsmedium, passagiert. Die Zellsuspension wird 5 Minuten bei 1000 UpM zentrifugiert, der Überstand abgesaugt und die Zellen in 6 ml Inkubationsmedium resuspendiert, auf 3 mittlere Zellkulturflaschen überführt und jeweils 10 ml Inkubationsmedium dazugegeben.

Am Tag 17 erfolgte ein drittes Passagieren auf insgesamt 6 mittlere Zellkulturflaschen und am Tag 24 ein viertes Passagieren auf insgesamt 12 mittlere Zellkulturflaschen. Spätestens jetzt waren alle primären Zellen bis auf die Stammzellen aus der Zellkultur entfernt.

Die Stammzellen können weiter kultiviert werden und so oft passagiert und ausgesät wie gewünscht. Das Aussäen erfolgt vorzugsweise jeweils in einer Dichte von 2-4 x 10⁵ Zellen/cm² in Inkubationsmedium.

### BEISPIEL 3

Die Isolierung und Kultivierung aus exokrinem Gewebe der Ohrspeicheldrüse eines Menschen erfolgte analog dem Pankreas-Protokoll mit den folgenden Abweichungen:
1. Das exokrine Gewebe der Ohrspeicheldrüse war eine Mischung von acinärem Gewebe und tubulösem Gewebe.
2. Nachdem Speicheldrüsen weniger Proteasen und Amylasen als Pankreas enthalten, ist es möglich, das Speicheldrüsengewebe vor der Aufarbeitung einige Zeit im Kühlschrank bei etwa 4°C aufzubewahren, ohne dass das Gewebe zu sehr geschädigt wird. Im konkreten Beispielsfall betrug die Aufbewahrungszeit 15 h und brachte keine nachteiligen Folgen für die Isolierung der gewünschten Stammzellen mit sich.

Das folgende Beispiel 4 beschreibt detailliert ein Arbeitsprotokoll zur Herstellung von organoiden Körpern.

### BEISPIEL 4

Die undifferenzierten Zellen werden mit einer Lösung aus 10 ml PBS, 4 ml Trypsin, 8 ml Differenzierungsmedium abtrypsiniert und 5 Minuten abzentrifugiert. Das resultierende Pellet wird so in Differenzierungsmedium resuspendiert, dass sich eine Verdünnung von 3000 Zellen je 100 µl Medium einstellt. Anschließend werden die Zellen nochmals gut mit einer 3 ml Pipette suspendiert.

Von bakteriologischen Petrischalen, die vorher mit jeweils 15 ml PBS (37 °C) pro Platte beschichtet worden sind, wird der Deckel abgenommen und umgedreht. Mit Hilfe einer automatischen Pipette werden auf einen Deckel ca. fünfzig 20 µl Tropfen gegeben. Der Deckel wird dann schnell umgedreht und auf die mit Differenzierungsmedium gefüllte Petrischale gegeben, sodass die Tropfen nach unten hängen. Die Petrischalen werden anschließend vorsichtig in den Brutschrank gestellt und für 48 h inkubiert.

Daraufhin werden die in den hängenden Tropfen aggregierten Zellen, die die organoiden Körper bilden, aus jeweils vier Deckeln in je eine bakteriologische Petrischale mit 5 ml Inkubationsmedium mit 20 % FKS überführt und für weitere 96 h kultiviert.

Die organoiden Körper werden nun vorsichtig mit einer Pipette aufgesammelt, und in mit 0,1 % Gelatine beschichtete Zellkulturgefäße mit Differenzierungsmedium überführt. In einer besonders bevorzugten Ausgestaltung des Verfahrens verwendet man als Kulturgefäß mit 0,1% Gelatine beschichtete 6 cm Petrischalen, in die 4 ml Differenzierungsmedium vorgelegt wurde und die anschließend mit je 6 organoiden Körpern beschickt werden. Ein weiteres bevorzugtes Kulturgefäß sind mit 0,1 % Gelatine beschichtete Chamber Slides, in die 3 ml Differenzierungsmedium vorgelegt wurde und die anschließend mit je 3-8 organoiden Körpern beschickt werden. Daneben können auch 24-Mulden-Mikrotiterplatten verwendet werden, die mit 0,1 % Gelatine beschichtet wurden und in die je 1,5 ml pro Mulde Differenzierungsmedium vorgelegt wurde und anschließend mit je 4 organoiden Körpern beschickt werden.

Derart kultiviert, ist die Differenzierungsfähigkeit der Zellen in den organoide Körper aktiviert und die Zellen können sich insbesondere in hormonproduzierenden Zellen differenzieren. Die Zellen können sowohl als organoide Körper als auch als einzelne Zellen gelagert und kultiviert werden und behalten ihre Pluripotenz.

### (II) Differenzierung in den organoiden Körpern und/oder Gewebekörpern

Nach der Isolierung und ggf. Aggregation liegen in den jeweiligen Zellkulturen oder organoiden Körpern die Stammzellen oder gegebenenfalls bereits spontan differenzierte, hormonproduzierende Zellen vor. Im weiteren Verfahren erfolgt die stimulierte Differenzierung der Stammzellen oder das stimulierte Wachstum der bereits differenzierten Zellen gemäß Schritt 200 (Figur 1). Hierzu werden eine oder mehrere der obengenannten Stimulationsbehandlungen durchgeführt. Die Stimulation der Differenzierung kann mit dem obengenannten Differenzierungsmedium (I) dahingehend erfolgen, dass allgemein eine Differenzierung der Stammzellen oder organoiden Körper stattfindet. Die Erfinder haben festgestellt, dass die Differenzierung neben anderen Zelltypen insbesondere die hormonproduzierenden Zellen ergibt, die dann bei der weiteren Bearbeitung (Schritt 300 in Figur 1) selektiert werden. Alternativ ist die Differenzierung mit den obengenannten weiteren Differenzierungsmedien vorgesehen, mit denen eine vorrangige Differenzierung hin zu den hormonproduzierenden Zellen erfolgt und bei deren Verwendung die nachfolgenden Schritte der Identifizierung und Selektion vereinfacht werden. Das nachfolgende Beispiel 5 bezieht sich auf die Stimulation mit dem Differenzierungsmedium (I), während die übrigen Beispiele sich auf die anderen Differenzierungsmedien und Stimulationstechniken beziehen.

### BEISPIEL 5

Für die Induktion der Differenzierung wurden vorzugsweise Stammzellen nach dem 42. Tag der Kultivierung verwendet. Die Verwendung von Stammzellen nach der 3. oder 4. Passage oder von Zellen, die bei der Temperatur von flüssigem Stickstoff 12-18 Monate lang gelagert worden waren, war ebenfalls problemlos möglich.

Zunächst wurden die Zellen im Differenzierungsmedium (I) mit der oben angegebenen Zusammensetzung überführt und auf eine Dichte von etwa 3 x 10⁴ Zellen/ml eingestellt; z.B. durch Trypsinbehandlung einer Stammzelkultur in Nährmedium, 5-minütige Zentrifugation bei 1000 UpM und Resuspendieren des Pellets im Differenzierungsmedium (I) und Verdünnung soweit erforderlich.

Anschließend wurden mit einer 20-µl Pipette ca. 50 20-µl-Tropfen (600 Zellen/20 µl) auf die Innenseite des Deckels einer bakteriologischen Petrischale gegeben (gestopfte Spitzen) und die Deckel vorsichtig auf die mit PBS gefüllten Petrischalen gestülpt, sodass die Tropfen nach unten hängen. Für jeden Deckel wurde eine neue Spitze verwendet. Die Petrischalen wurden anschließend vorsichtig in den Brutschrank gestellt und 48 h lang bei 37°C inkubiert.

Danach wurden die in den hängenden Tropfen aggregierten Zellen, die organoide Körper, aus jeweils vier Deckeln in je eine bakteriologische Petrischale mit 5 ml Inkubationsmedium mit 20 % FKS überführt (Deckel schräg halten und die organoiden Körper mit etwa 2,5 ml Nährmedium abspülen) und für weitere 5-9 Tage, vorzugsweise 96 h, kultiviert.

Die organoiden Körper wurden nun vorsichtig mit einer Pipette aufgesammelt und in mit 0,1 % Gelatine beschichtete Zellkulturgefäße mit Differenzierungsmedium (I) überführt. Die organoiden Körper vermehrten sich nun und wuchsen in zum Teil einzelnen Zellkolonien, die wieder vermehrt vereinzelt und vermehrt werden konnten. In einer besonders bevorzugten Ausgestaltung des Verfahrens wurden als Kulturgefäß mit 0,1 % Gelatine beschichtete 6 cm Petrischalen verwendet, in die 4 ml Differenzierungsmedium (I) vorgelegt worden war, und diese mit je 6 organoiden Körpern beschickt. Ein weiteres bevorzugtes Kulturgefäß waren mit 0,1 % Gelatine beschichtete Chamber Slides, in die 3 ml Differenzierungsmedium vorgelegt wurde und die anschließend mit je 3-8 organoiden Körpern beschickt wurden und Thermanox-Platten (Nalge Nonc International, USA) für elektronenmikroskopische Studien. Ein weitere Alternative waren 24-Mulden-Mikrotiterplatten, die mit 0,1 % Gelatine beschichtet wurden und in die je 1,5 ml pro Mulde Differenzierungsmedium (I) vorgelegt wurde und die anschlie-βend mit je 4 organoiden Körpern beschickt wurden.

In einer bevorzugten Ausgestaltung des Verfahrens wurden organoide Körper etwa 7 Wochen lang in den gelatinebeschichteten 6-cm-Petrischalen kultiviert und danach wurden einzelne organoide Körper mit dem Microdissector (Eppendorf, Hamburg, Deutschland nach den Anweisungen des Herstellers ausgeschnitten und dann z.B. auf frische 6-cm-Petrischalen, Chamber Slides oder Thermanox-Platten überführt.

### BEISPIEL 6

Die Stimulation mit den oben genannten weiteren Differenzierungsmedien erfolgt analog.

### BEISPIEL 7

Figur 6 illustriert die Stimulation (Schritt 200) einer Stammzelle 1 auf einem Substrat 21 durch molekulare Signal- oder Differenzierungsfaktoren 5, die im Kultivierungsmedium enthalten sind. Auf der Oberfläche der Stammzelle 1 befinden sich Zellrezeptoren 1a. Beim Ankoppeln der Signal- oder Differenzierungsfaktoren an den Zellrezeptoren 1a erfolgt eine Prägung der Stammzelle 1 und der Beginn der Differenzierung hin zur gewünschten Hormon produzierenden Zelle.

Die molekularen Signal- oder Differenzierungsfaktoren 5 umfassen an sich bekannte biologische Makromoleküle, Zellbestandteile von pankreatischen Zellen oder insbesondere Zellen, Zellbestandteile oder Zellgruppen verwendet werden, die im endokrinen Gewebe der Pankreas in der Umgebung der Langerhans'schen Inseln angeordnet sind

### BEISPIEL 8

Figur 7 illustriert entsprechend die Prägung einer Stammzelle 1 mit einer bereits differenzierten Zelle 6, auf deren Membranoberfläche entsprechende Signal- oder Differenzierungsfaktoren fixiert oder natürlich gegeben sind.

### BEISPIEL 9

Gemäß einer weiteren Variante kann eine Genaktivierung der Stammzellen vorgesehen sein, wie sie beispielsweise in DE 102 90 025 T1 beschrieben ist.

### BEISPIEL 10

Figur 8 illustriert die Insulinproduktion der differenzierten Zellen mit einer mikroskopischen Aufnahme von Insulin produzierenden Zellen, die aus exokrinem Pankreas des Menschen gewonnen wurden. Entsprechende Ergebnisse sind mit Speicheldrüsen des Menschen oder von anderen Wirbeltieren gefunden worden. Die mit den Pfeilen markierten Punkte repräsentieren das von den Zellen produzierte Insulin. Der weiße Strich entspricht einer Länge von 20 µm im Original.

Die in der vorstehenden Beschreibung, den Ansprüchen und den Zeichnungen offenbarten Merkmale der Erfindung können sowohl einzeln oder auch in Kombination für die Verwirklichung der Erfindung in ihren verschiedenen Ausgestaltungen von Bedeutung sein.

## Patentansprüche

1. Verfahren zur Bildung von pankreatisches Hormon produzierenden Zellen, umfassend:
- eine Kultivierung und Differenzierung von pluripotenten Stammzellen, die aus differenziertem exokrinen Drüsengewebe eines Organismus gewonnen wurden und das Vermögen zur Bildung von organoiden Körpern aufweisen.

2. Verfahren nach Anspruch 1, bei dem primär aus dem Organismus isolierte Stammzellen kultiviert und differenziert werden.

3. Verfahren nach Anspruch 1, bei dem eine Aggregation der Stammzellen zu organoiden Körpern vorgesehen ist.

4. Verfahren nach Anspruch 3, bei dem die Differenzierung der Stammzellen in den organoiden Körpern erfolgt.

5. Verfahren nach Anspruch 3, bei dem sekundär aus den organoiden Körpern isolierte Stammzellen kultiviert und differenziert werden.

6. Verfahren nach mindestens einem der vorhergehenden Ansprüche, bei dem eine Stimulation der Bildung der pankreatisches Hormon produzierenden Zellen vorgesehen ist, die eine stimulierte Vermehrung der pankreatisches Hormon produzierende Zellen und/oder eine stimulierte Differenzierung der Stammzellen umfasst.

7. Verfahren nach Anspruch 6, bei dem die Stimulation mindestens eine der folgenden Stimulationsbehandlungen umfasst:
- Behandlung mit Überständen einer Primärkultur des endokrinen Pankreas,
- Behandlung mit Überständen von Zelllinien des endokrinen Pankreas,
- Co-Kultur mit differenzierten Zellen des endokrinen Pankreas,
- Co-Kultur mit Zelllinien des endokrinen Pankreas, oder
- Behandlung mit immobilisierten molekularen Wachstumsfaktoren,
- Aktivierung von mindestens einem Gen, das in die Differenzierung der Stammzellen zu den pankreatisches Hormon produzierenden Zellen involviert ist, und
- Behandlung mit in einer Flüssigkeit gelösten molekularen Wachstumsfaktoren.

8. Verfahren nach Anspruch 7, bei dem die Behandlung mit immobilisierten molekularen Wachstumsfaktoren eine Zellprägung mit molekularen, auf einem Träger immobilisierten Differenzierungsfaktoren umfasst.

9. Verfahren nach Anspruch 8, bei dem als Träger ein synthetisches Substrat, eine Zellmembran oder ein dreidimensionales Matrixsubstrat verwendet wird.

10. Verfahren nach mindestens einem der vorhergehenden Ansprüche, bei dem eine Identifizierung und Selektion der pankreatisches Hormon produzierenden Zellen vorgesehen ist.

11. Verfahren nach Anspruch 10, bei dem die Selektion der pankreatisches Hormon produzierende Zellen ein Zellsortierverfahren umfasst.

12. Verfahren nach Anspruch 10 oder 11, bei dem nicht identifizierte und selektierte Zellen einer weiteren Kultivierung und Differenzierung unterzogen werden.

13. Verfahren nach mindestens einem der vorhergehenden Ansprüche, bei dem Stammzellen verwendet werden, die aus sekretorischen Drüsen oder Drüsen des gastro-intestinalen Traktes des Organismus gewonnen wurden.

14. Verfahren nach Anspruch 13, bei dem Stammzellen verwendet werden, die aus der Pankreas oder der Speicheldrüse des Organismus gewonnen wurden.

15. Verfahren nach mindestens einem der vorhergehenden Ansprüche, bei dem Stammzellen aus Drüsengewebe verwendet werden, das acinäres Gewebe ist.

16. Verfahren nach mindestens einem der vorhergehenden Ansprüche, bei dem Stammzellen von einem Wirbeltier, vorzugsweise einem Säuger verwendet werden.

17. Verfahren nach Anspruch 16, bei dem Stammzellen von einem Primaten, insbesondere einem Menschen verwendet werden.

18. Verfahren nach mindestens einem der vorhergehenden Ansprüche, bei dem die pankreatisches Hormon produzierenden Zellen Insulin produzieren.

## Claims

1. A process for the generation of cells producing pancreatic hormone, comprising:
- a cultivation and differentiation of pluripotent stem cells obtained from differentiated exocrine glandular tissue of an organism and having the capability to form organoid bodies.

2. The process according to Claim 1, in which stem cells isolated primarily from the organism are cultivated and differentiated.

3. The process according to Claim 1, in which an aggregation of the stem cells to organoid bodies is provided.

4. The process according to Claim 3, in which the differentiation of the stem cells is carried out in the organoid bodies.

5. The process according to Claim 3, in which stem cells isolated secondarily from the organoid bodies are cultivated and differentiated.

6. The process according to at least one of the preceding claims, in which a stimulation of the generation of cells producing pancreatic hormone that comprises a stimulated propagation of the cells producing pancreatic hormone and/or a stimulated differentiation of the stem cells is provided.

7. The process according to Claim 6, in which the stimulation comprises at least one of the following stimulation treatments:
- treatment with supernatants of a primary culture of the endocrine pancreas,
- treatment with supernatants of cell lines of the endocrine pancreas,
- co-culture with differentiated cells of the endocrine pancreas,
- co-culture with cell lines of the endocrine pancreas, or
- treatment with immobilized molecular growth factors,
- activation of at least one gene that is involved in the differentiation of stem cells into the cells producing pancreatic hormone, and
- treatment with molecular growth factors dissolved in a liquid.

8. The process according to Claim 7, in which the treatment with immobilized molecular growth factors comprises a cellular imprinting with molecular differentiation factors immobilized on a support.

9. The process according to Claim 8, in which a synthetic substrate, a cell membrane or a three-dimensional matrix substrate is used as support.

10. The process according to at least one of the preceding claims, in which an identification and selection of the cells producing pancreatic hormone is provided.

11. The process according to Claim 10, in which the selection of the cells producing pancreatic hormone comprises a cell sorting process.

12. The process according to Claim 10 or 11, in which non-identified and selected cells are subjected to a further cultivation and differentiation.

13. The process according to at least one of the preceding claims, in which stem cells that were obtained from secretory glands or glands of the gastrointestinal tract of the organism are used.

14. The process according to Claim 13, in which stem cells that were obtained from the pancreas or the salivary gland of the organism are used.

15. The process according to at least one of the preceding claims, in which stem cells from glandular tissue that is acinar tissue are used.

16. The process according to at least one of the preceding claims, in which stem cells from a vertebrate, preferably a mammal, are used.

17. The process according to Claim 16, in which stem cells from a primate, especially a human being, are used.

18. The process according to at least one of the preceding claims, in which the cells producing pancreatic hormone produce insulin.

## Revendications

1. Procédé pour la formation de cellules productrices de l'hormone pancréatique, comprenant :
- une culture et une différentiation de cellules souches pluripotentes qui ont été extraites d'un tissu glandulaire exocrine différencié d'un organisme et qui présentent la capacité de formation de corps organoïdes.

2. Procédé selon la revendication 1, dans lequel des cellules souches isolées hors de l'organisme sont d'abord cultivées et différenciées.

3. Procédé selon la revendication 1, dans lequel il est prévu une agrégation des cellules souches pour former des corps organoïdes.

4. Procédé selon la revendication 3, dans lequel la différentiation des cellules souches se fait dans les corps organoïdes.

5. Procédé selon la revendication 3, dans lequel des cellules souches isolées hors des corps organoïdes sont ensuite cultivées et différenciées.

6. Procédé selon au moins l'une quelconque des revendications précédentes, dans lequel il est prévu une stimulation de la formation des cellules productrices de l'hormone pancréatique qui comprend une multiplication stimulée des cellules productrices de l'hormone pancréatique et/ou une différentiation stimulée des celles couches.

7. Procédé selon la revendication 6, dans lequel la stimulation comprend au moins l'un des traitements de stimulation suivants :
- traitement avec surnageant de lignées cellulaires du pancréas endocrinien,
- co-culture avec des cellules différenciées du pancréas endocrinien,
- co-culture avec des lignées cellulaires du pancréas endocrinien, ou
- traitement avec des facteurs de croissance moléculaires immobilisés,
- activation d'au moins un gène qui est impliqué dans la différentiation des cellules couches en cellules productrices de l'hormone pancréatique, et
- traitement avec des facteurs de croissance moléculaires dissous dans un liquide.

8. Procédé selon la revendication 7, dans lequel le traitement avec des facteurs de croissance moléculaires immobilisés comprend une empreinte cellulaire avec des facteurs de différentiation moléculaires immobilisés sur un support.

9. Procédé selon la revendication 8, dans lequel on utilise comme support un substrat synthétique, une membrane cellulaire ou un substrat matriciel tridimensionnel.

10. Procédé selon au moins l'une des revendications précédentes, dans lequel il est prévu une identification et une sélection des cellules productrices de l'hormone pancréatique.

11. Procédé selon la revendication 10, dans lequel la sélection des cellules productrices de l'hormone pancréatique comprend un procédé de tri des cellules.

12. Procédé selon la revendication 10 ou 11, dans lequel les cellules non identifiées et sélectionnées sont soumises à une culture et différentiation ultérieure.

13. Procédé selon au moins l'une des revendications précédentes, dans lequel on utilise des cellules souches qui ont été extraites de glandes sécrétoires ou de glandes de l'appareil gastro-intestinal de l'organisme.

14. Procédé selon la revendication 13, dans lequel on utilise des cellules souches qui ont été extraites du pancréas ou de la glande salivaire de l'organisme.

15. Procédé selon au moins l'une des revendications précédentes, dans lequel on utilise des cellules souches provenant d'un tissu glandulaire qui est un tissu acinaire.

16. Procédé selon au moins l'une des revendications précédentes, dans lequel on utilise des cellules souches d'un vertébré, de préférence d'un mammifère.

17. Procédé selon la revendication 16, dans lequel on utilise des cellules souches d'un primate, en particulier d'un homme .

18. Procédé selon au moins l'une des revendications précédentes, dans lequel les cellules productrices de l'hormone pancréatique produisent de l'insuline.
